Europäisches Patentamt

European Patent Office

Office européen des brevets

(19)

(11) Publication number: **0 001 247**
**A1**

(12) **EUROPEAN PATENT APPLICATION**

(21) Application number: 78100854.5

(22) Date of filing: 11.09.78

(51) Int. Cl.²: **A 61 K 9/18**
**A 61 K 31/44**

(30) Priority: 14.09.77 JP 110812/77
19.09.77 JP 112331/77

(43) Date of publication of application:
04.04.79 Bulletin 79/7

(84) Designated contracting states:
BE DE FR GB NL

(71) Applicant: KANEBO LTD.
17-4 sumida 5-chome sumida-ku
Tokyo(JP)

(72) Inventor: Sugimoto, Isao
7-7 Saidaiji-Kunimicho 2-chome
Nara(JP)

(72) Inventor: Kuchiki, Akira
13-11 Senriokahigashi Settsu-shi
Osaka(JP)

(74) Representative: Reitstötter, Josef et al,
Bauerstrasse 22 P.O. Box 780
D-8000 München 43(DE)

(54) Pharmaceutical preparation containing nifedipine and a method for producing the same.

㉟ Stable, easily absorbable preparations of nifedipine suitable for oral administration are provided comprising a solution of nifedipine in polyethylene glycol carried on pharmaceutically acceptable porous carrier or a noncrystalline dispersion of nifedipine in polyvinylpyrrolidone.

EP 0 001 247 A1

Croydon Printing Company Ltd

Pharmaceutical Preparation containing Nifedipine

This invention relates to easily absorbable pharmaceutical preparations of dimethyl 1,4-dihydro-2,6-dimethyl-4-(2-nitrophenyl)-3,5-pyridinedicarboxylate (hereinafter referred to as "nifedipine") for oral administration.

Angina pectoris caused by coronary occlusion needs administration of a vasodilator which can exhibit its pharmacological activity within few minutes after the administration.

Nifedipine known as an excellent remedy for the treatment of angina pectoris has a disadvantage that it is not sufficiently soluble in water and hence not absorbed rapidly in the body. Conventional dosage forms thereof for oral administration such as tablets, granules in the form of a mixture with conventional pharmaceutical excipients do not afford rapid onset of their pharmacological activity.

In order to improve the absorbability, nifedipine is dissolved in polyethylene glycol and the solution is encapsulated to give soft capsules (Japanese Patent Kokai 48-28621). The soft capsules of this type have been commercially available and widely used. Because of difficulty in the encapsulating technique, the capsules have to be of relatively large sizes, for instance, about 600mg/capsule, which are often difficult to be swallowed by the patients. Moreover nifedipine is very unstable upon exposure to light in a solution. It is necessary, therefore, for the soft capsules of the above-mentioned type to add a UV light absorbing agent in the walls thereof for the prevention of photochemical decomposition of the active ingredient on storage. One of the disadvantages of soft capsules is that their production costs are relatively high.

Accordingly it is an object of the present invention to provide a pharmaceutical preparation of nifedipine for oral administration which is easily absorbable and stable, and smaller in size than the soft capsules now available.

Another object of the present invention is to provide a pharmaceutical preparation which is compatible with conventional excipients for producing tablets granules and the like without impairing its absorbability, stability and other charcteristics.

According to a first preferred embodiment of the present invention, these and other objects and advantages are accomplished by providing a pharmaceutical preparation comprising a solution of nifedipine/in polyethylene glycol carried on a pharmaceutically acceptable porous carrier substance in such a manner that the increase in the resting angle of the resulting preparation is not greater than 5° as compared with that of the carrier substance.

The polyethylene glycols which may be used for dissolving nifedipine/occur as liquid phase at room temperature and have an average molecular weight from 200 to 600. Since the higher concentration of nefedipine in the polyethylene glycols is preferable for obtaining smaller, easily swallowable dosage forms, it is advantageous to dissolve nifedipine in polyethylene glycol as much as possible i.e. near saturation. For example, 1 part by weight of nifedipine dissolves in 20 parts by weight of polyethylene glycol having an average molecular weight of 400.

In accordance with the present invention, the above solution is adsorbed on a pharmaceutically acceptable porous carrier substance. Examples of such carrier substances include synthetic aluminum silicate, dry aluminum hydroxide, magnesium aluminometasilicate, calcium lactate, low-density silicic anhydride, microcrystalline cellulose and the like. They must occur as free-flowing, fine particles. The solution of nifedipine in polyethylene glycol may be carried on the carrier substance by mixing the solution and the carrier in conventional manner. The ratio of the solution to the carrier substance is such that increase in the resting angle of the resulting mixture is not greater than 5° as compared with that of the carrier substance. The resting angle of each original carrier substance (i.e. a maximum angle of inclination at which a pile of a powdery substance can rest without flowing down and spreading) normally increases with increase in the quantity of the polyethylene glycol solution to be mixed therewith. Within said range of increase in the resting angle, the free-flowing property of the original carrier substance will be retained to a suitable extent for further processing and the polyethylene glycol solution may be adsorbed and carried in fine interstices present on the exterior surface of the carrier substance.

It is surprising that nifedipine in this mixture is more stable against photochemical degradation than in the solution in polyethylene glycol alone while the absorbability of said solution has not been impaired by mixing with said carrier substances.

The amount of the polyethylene glycol solution to be mixed with the carrier substance is preferably as much as possible within the aforementioned limit to minimize the size of the finished dosage forms. The specific values of the preferred ratio of the solution to the carrier substances vary with the molecular weight of polyethylene glycols and the particular carrier substances but may be easily determined by a simple preliminary test. For example, a solution consisting of 10 parts by weight of nifedipine and 200 parts by weight of polyethylene glycol (average M.W.=400) may be mixed with about 100 parts by weight of low density silicic anhydride or about 200 parts by weight of microcrystalline cellulose.

According to another preferred embodiment of the present invention, there is provided a pharmaceutical preparation comprising nifedipine dispersed and/or dissolved in 0.5 to 30 times, preferably from 2 to 5 times of polyvinylpyrrolidone (hereinafter referred to as "PVP") based on the weight of nifedipine in substantially noncrystalline state. Nifedipine is practically stable in this state against photochemical degradation and may be rapidly absorbed into the body through digestive canals. The dispersion or solid solution may be pulverized or shaped into particles of various sizes in conventional manner and used for the production of various dosage preparations such as tablets or granules.

The noncrystalline dispersion or solid solution may be produced by dissolving nifedipine and PVP in a common solvent and removing the solvent. Examples of the solvent include acetone, chloroform, methanol, ethanol and dichloromethane. Ethanol is most preferable because of high solubility of the solutes therein and low toxicity of residual solvent or vapor thereof.

The molecular weight of PVP is not critical but preferably ranges from 10,000 to 50,000 because the capability of preventing crystallization of nifedipine and the solubility in the solvent are well balanced. As aforementioned, at least 0.5 times of PVP based on the weight of nifedipine are needed for effectively preventing crystallization of nifedipine in the resulting dispersion and more than 30 times of PVP are not desirable because of excessive bulkiness. A ratio of PVP to nifedipine between 2 to 5 times is generally preferable. Removal of the solvent may be carried out by the conventional evaporation technique either under normal or a reduced pressure. Alternatively the solution of nifedipine and PVP in a common solvent may be spray-dried or dried during granulating or tableting stage after mixing the solution with other pharmaceutical excipients.

Thus the solution and the excipient are kneaded and the resulting mass may be shaped into granules of suitable size and then dried. The dry granules may also be produced by spraying the solution onto the nuclei of the expcipients in a fluid bed while blowing hot air.

The resulting particles or granules are preferably pulverized to improve the solubility in water or digestive juice and also to improve the mixing property with other drug additives.

Since the solution of nifedipine and PVP is unstable to light, care should be taken not to expose the solution to light excessively during · · processing the solution. Once the solution has been processed into the powder form in accordance with the present invention, it becomes stable for a long period of time.

The powder or granules made in accordance with the presention invention may be further processed with other drug additives such as excipients, disintegrating agents or lubricants for producing pharmaceutical preparations such as tablets, granules, pearls or buccal tablets or they may be directly filled in hard capsules. Normally they contain 10mg of nifedipine per unit and are administered three times per day at a dose of 10mg in terms of nifedipine for the treatment of angina pectoris or other diseases. The dose may vary depending upon particular patients and conditions.

The present invention is more fully illustrated by the following examples. These examples are included here for the purpose of illustration only and are not intended as a limitation.

## Example 1

10g of nifedipine was dissolved in 200g of polyethylene glycol having an average molecular weight of 400. The resulting solution was mixed with 100g of magnesium aluminometasilicate (60-80 mesh). The resulting mixture was filled in #2 hard capsules in an amount of 310mg per capsule. Each capsule contains 10mg of nifedipine.

## Example 2

10g of nifedipine was dissolved in 200g of polyethylene glycol having an average molecular weight of 300. The resulting solution was mixed with 100g of low density silicic anhydride.

The resulting mixture was then mixed with 160g of lactose and 30g of talc to give powder. 500mg of the resulting powder contains 10mg of nifedipine.

- 8 -

## Example 3

10g of nifedipine was dissolved in 190g of polyethylene glycol having an average molecular weight of 400. The solution was mixed with 100g of synthetic aluminum silicate. The mixture was kneaded with 170g of dry corn strach and corn starch paste (dry content 20g). The resulting mass was extruded through a mesh screen and shaped into granules. The granules were dried at 40℃ for 5 hours and 10g of magnesium stearate was added thereto. The product was then sieved.

## Example 4

10g of nifedipine and 30g of PVP (average molecular weight = 40,000) were dissolved in 200ml of ethanol with stirring. The solution was then spray-dried. The resulting powder was mixed with 20g of microcrystalline cellulose, 19g of calcium salt of CMC and 1g of magnesium stearate. The mixture was compressed into tablets each weighing 80mg and having a diameter of 6mm. This tablet contains 10mg of nifedipine.

## Example 5

The solution obtained in Example 4 was kneaded with 450g of lactose. The resulting mass was extruded through 24 mesh screen and shaped into granules. The granules were dried at 40℃ and 10g of talc was added thereto. 500mg of this product contains 10mg of nifedipine.

## Example 6

In a conventional fluid bed pelletizing machine 100g of lactose was charged as nuclei and the solution obtained in Example 4 was sprayed thereon while drying. The resulting fine granules were filled in #3 hard capsules in an amount of 140mg per capsule. Each capsule contains 10mg of nifedipine.

## Example 7

10g of nifedipin and 20g of PVP (average molecular weight = 40,000) were dissolved in 200ml of ethanol with stirring. The resulting solution was evaporated in vacuo to remove the solvent. The residue was pulverized.

The resulting powder was processed in the same manner as Example 4 to obtain tablets each weighing 70mg and containing 10mg of nefedipine.

## Example 8

Example 7 was repeated except that 200ml of methanol were replaced for 200ml of ethanol. The resulting powder was kneaded with 50g of dry corn starch, 60g of lactose and starch paste (dry content 18g). The resulting mass was shaped into granules and dried. The granules were mixed with 2g of magnesium stearate and the mixture was compressed into buccal tablets each weighing 160mg and containing 10mg of nifedipine.

## Dissolving Test

The products obtained in the above examples were tested on their solubilities in artificial gastric juice in comparison with controls.

Control No. 1 is a commercially available soft capsule of nifedipine and Control No. 2 is a compressed tablet produced from crystallin powder of nifedipine by a conventional manner.

500ml of artificial gastric juice (Solution No. 1, Japanese Pharmacopeia) was placed in a 1 litre beaker and stirred with a stirrer having four blades at 150 r.p.m. at 37℃. To this solution was added each test sample in an amount equivalent to 10mg of nifedipine. The amount of nifedipine which was dissolved in the artificial gastic juice was determined spectrometrically at the intervals as indicated in Table 1.

The results obtained are shown in Table 1 in terms of per cent of dissolved nifedipine versus added nifedipine.

Table 1

| | | Time (min.) | | | | |
|---|---|---|---|---|---|---|
| | Sample | 1 | 2 | 4 | 6 | 10 |
| | Ex. 1 | 0 | 38 | 85 | 101 | 104 |
| | Ex. 2 | 17 | 52 | 94 | 98 | 99 |
| | Ex. 3 | 11 | 33 | 95 | 98 | 98 |
| | Ex. 4 | 5 | 18 | 97 | 101 | 98 |
| | Ex. 5 | 14 | 44 | 103 | 99 | 101 |
| | Ex. 6 | 0 | 20 | 88 | 102 | 103 |
| | Ex. 7 | 8 | 26 | 92 | 100 | 97 |
| | Ex. 8 | 22 | 49 | 98 | 101 | 99 |
| | Control 1 | 0 | 25 | 100 | 98 | 102 |
| | Control 2 | 2 | 5 | 11 | 15 | 17 |

Remarks: Control 2 was prepared by compressing a mixture consisting of 10g of nifedipine, 38g of lactose, 30g of microcrystalline cellulose and 2g of magnesim stearate in conventional manner.

Each tablet (80mg) contains 10mg of nifedipine.

As will be apparent from Table 1, the preparations of the present invention exhibit good dissolving characteristics as the commercial soft capsules, and are dissolved more rapidly than the tablets produced by conventional technique. Some of the products (Examples 2, 3, 4, 7 and 8) are excellent in the initial dissolving rate.

0001247

## Blood Level

The products of Examples 1, 4 and 5 and Controls 1 and 2 were orally administered to respective groups of five beagle dogs (3 male, 2 female) at a dose equivalent to 20mg of nifedipine per animal. Concentrations of nifedipine/in the blood in terms of µg/ml were measured at the intervals as indicated in Table 2.

### Table 2

| Sample | Time (hour) | | | | |
| --- | --- | --- | --- | --- | --- |
| | 0.5 | 1 | 2 | 3 | 5 |
| Ex. 1 | 0.55 | 0.55 | 0.26 | ND | ND |
| Ex. 4 | 0.69 | 0.50 | 0.20 | 0.17 | ND |
| Ex. 5 | 0.60 | 0.45 | 0.25 | ND | ND |
| Control 1 | 0.60 | 0.51 | 0.22 | 0.15 | ND |
| Control 2 | 0.23 | 0.33 | 0.27 | 0.20 | ND |

Remarks: Mean value of 5 animals. ND means "not detected".

From the foregoing results it will be understood that the blood level of the administered preparations of this invention rises rapidly as the commercial product (Control 1) and the tablets produced by conventional technique (Control 2) are less absorbable as represented by the delayed low peak.

It is to be understood that the foregoing detailed description is given merely by way of illustration and that many variations may be made therein without departing from the spirit of the present invention.

- 1 -

0001247

WHAT IS CLAIMED IS:

1. An easily absorbable pharmaceutical preparation for oral administration comprising a solution of dimethyl 1,4-dihydro-2,6-dimethyl-4-(2-nitrophenyl)-3,5-pyridinedi-carboxylate in polyethylene glycol carried on a pharmaceutically acceptable porous carrier substance in such a manner that the increase in the resting angle of the resulting preparation is not greater than 5° as compared with that of the carrier substance.

2. The preparation of Claim 1, wherein said polyethylene glycol has an average molecular weight from 200 to 600.

3. The preparation of Claim 1, wherein said carrier substance is selected from the group consisting of synthetic aluminum silicate, dry aluminum hydroxide, magnesium aluminometasilicate, calcium lactate, low-density silicic anhydride and microcrystalline cellulose.

4. An easily absorbable pharmaceutical preparation for oral administration comprising dimethyl 1,4-dihydro-2,6-dimethyl-4-(2-nitrophenyl)-3,5-pyridinedicarboxylate dispersed and / or dissolved in 0.5 to 30 times of polyvinylpyrrolidone based on the weight of said pyridine-dicarboxylate in a substantially noncrystalline state.

5. The preparation of Claim 4, wherein said polyvinylpyrrolidone has an average molecular weight of 10,000 to 50,000.

6. The preparation of Claim 4, wherein the ratio of polyvinylpyrrolidone to said pyridinedicarboxylate ranges from 2 to 5.

7. A method for producing the pharmaceutical preparation of Claim 4 which comprises dissolving dimethyl 1,4-dihydro-2,6-dimethyl-4-(2-nitrophenyl)-3,5-pyridinedicarboxylate and from 0.5 to 30 times thereof of polyvinylpyrrolidone in their common solvent and removing the solvent from the solution.

8. The method of Claim 7, wherein said solvent is selected from the group consisting of acetone, chloroform, methanol, ethanol and dichloromethane.

0001247

## European Patent Office

### EUROPEAN SEARCH REPORT

Application number

EP 78 10 0854

| Category | DOCUMENTS CONSIDERED TO BE RELEVANT | Relevant to claim | CLASSIFICATION OF THE APPLICATION (Int. Cl.²) |
|---|---|---|---|
| | Citation of document with indication, where appropriate, of relevant passages | | A 61 K 9/18 31/44 |
| X | DE – A – 2 400 819 (BAYER)  * Page 2, line 12 – page 3, line 25; examples; claims * | 1–8 | |
| | DE – A – 2 546 371 (SANDOZ)  * Page 2, line 1 to page 5, line 14; claims * | 1–8 | |
| A | BE – A – 621 147 (AMERICAN VISCOSE) | 3 | |
| A | US – A – 3 673 163 (WALKLING) | 4–8 | |

TECHNICAL FIELDS SEARCHED (Int.Cl.²)

A 61 K 9/18
9/14
9/48
31/44

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 29–11–1978 | JONAS |

EPO Form 1503.1   06.78